# EUROPEAN PATENT APPLICATION

(11) **EP 2 644 100 A1**
(43) Date of publication of application: **02.10.2013**
(21) Application number: 13160621.2
(22) Date of filing: 22.03.2013
(51) Int. Cl.: A61B 8/00

(54) **Ultrasound diagnosis apparatus and operating method thereof**

(30) Priority: 27.03.2012 KR 20120031215
(71) Applicant: Samsung Medison Co., Ltd., Gangwon-do 250-870 (KR)
(72) Inventor: Hyun, Dong-gyu, Gangwon-do (KR); Shinozuka, Norio, Gangwon-do (KR)
(74) Representative: Lorenz, Markus

(57) **Abstract**

An ultrasound diagnosis apparatus and a method of operating the ultrasound diagnosis apparatus. The ultrasound diagnosis apparatus includes a probe for transmitting an ultrasound signal to an object and receiving a response signal from the object; a detector for detecting a sensor signal including at least one of location information representing a three-dimensional location of the probe and orientation information representing an orientation of the probe on the object; a storage unit for storing the sensor signal associated with the response signal; and an image processor for forming an ultrasound image based on the received response signal, forming an image indicator based on the sensor signal stored in the storage unit, and forming a display image including the ultrasound image and the image indicator corresponding to the ultrasound image.

## Description

### CROSS-REFERENCE TO RELATED PATENT APPLICATION

This application claims the benefit of Korean Patent Application No. 10-2012-0031215, filed on March 27, 2012, in the Korean Intellectual Property Office, the disclosure of which is incorporated herein in its entirety by reference.

### BACKGROUND

The present disclosure relates to an ultrasound diagnosis apparatus and a method of operating the ultrasound diagnosis apparatus.

Some conventional ultrasound diagnosis apparatuses acquire an ultrasound image by scanning an object and output the ultrasound image in real-time. In addition, the ultrasound diagnosis apparatus may store a data signal for outputting the ultrasound image and then may output the ultrasound image based on the stored data signal. The ultrasound image may be used to diagnose the object. However, a user of a conventional ultrasound diagnosis apparatus may not intuitively recognize a scanning angle and a scanning position of the object in the conventional method.

Therefore, a need exists for an ultrasound diagnosis apparatus and an operating method of the ultrasound diagnosis apparatus by which a user may intuitively recognize a scanning manner of an object.

### SUMMARY

The present disclosure provides an ultrasound diagnosis apparatus that allows a user thereof to intuitively recognize the state of scanning an object, and an operating method of the ultrasound diagnosis apparatus.

According to an aspect of the present disclosure, there is provided an ultrasound diagnosis apparatus including: a probe configured to transmit an ultrasound signal to an object and receiving a response signal from the object; a detector configured to detect a sensor signal including at least one of location information representing a three-dimensional location of the probe and orientation information representing a directivity of the probe on the object; a storage unit configured to store the sensor signal associated with the response signal; and an image processor configured to form an ultrasound image based on the received response signal, form an image indicator based on the sensor signal stored in the storage unit, and form a display image including the ultrasound image and the image indicator corresponding to the ultrasound image.

The ultrasound diagnosis apparatus may further include an output unit configured to output the display image.

The location information may represent three-dimensional coordinate values x, y, and z, and the orientation information represents a three-dimensional angle.

The storage unit may be further configured to store a data signal acquired based on the response signal, and the image processor may be further configured to form the ultrasound image by using the data signal stored in the storage unit and form the image indicator by using the sensor signal stored in the storage unit.

The data signal may include N frame data signals about N frames and the sensor signal may include M sensor sub-signals, each of the N frame data signals may correspond to one of the M sensor sub-signals, and N and M may be positive natural numbers.

The display image may include N frame display images of the N frames, and an n-th frame display image of an n-th frame among the N frame display images may include an n-th frame ultrasound image and an n-th frame image indicator. In the present disclosure, n may be any positive natural number from 1 to N, (e.g., n=1, 2,..., N).

The image processor may be further configured to form the n-th frame ultrasound image based on the n-th frame data signal stored in the storage unit, and form the n-th frame image indicator based on a sensor sub-signal corresponding to the n-th frame data signal among the M sensor sub-signals stored in the storage unit.

The storage unit may be further configured to store relational information indicating correspondence relations between the N frame data signals and the M sensor sub-signals.

The relational information may be frame information included in each of the M sensor sub-signals, or the storage unit may be further configured to store the N frame data signals, the M sensor sub-signals, and an information table indicating the relational information.

The image indicator may be a marker representing the location information and the orientation information of the probe with respect to the object by using at least one of an image and a text.

According to another aspect of the present disclosure, there is provided an method of operating an ultrasound diagnosis apparatus, the method including: transmitting an ultrasound signal from a probe to an object, and receiving a response signal from the object; detecting a sensor signal including at least one of location information representing a three-dimensional location of the probe and orientation information representing a directivity of the probe on the object; storing the sensor signal associated with the response signal; and forming an ultrasound image based on the received response signal, forming an image indicator based on the stored sensor signal, and forming a display image including the ultrasound image and the image indicator.

According to another aspect of the present disclosure, there is provided an ultrasound diagnosis apparatus including: a probe configured to transmit an ultrasound signal to an object and receive a response signal from the object; a detector configured to detect at least one of location information representing a three-dimensional location of the probe and orientation information representing a directivity of the probe on the object; and a storage unit configured to store the at least one of the location information and the orientation information associated with the received response signal.

According to another aspect of the present disclosure, there is provided a method of operating an ultrasound diagnosis apparatus, the method including: transmitting an ultrasound signal from a probe to an object, and receiving a response signal from the object; detecting at least one of location information representing a three-dimensional location of the probe and orientation information representing a directivity of the probe on the object; and storing the at least one of the location information and the orientation information associated with the response signal.

According to another aspect of the present disclosure, there is provided a non-transitory computer readable recording medium that, when executed, cause one or more computer processors to implement a program for executing a method of operating the ultrasound diagnosis apparatus.

According to another aspect of the present disclosure, there is provided an ultrasound diagnosis apparatus including: a probe configured to transmit an ultrasound signal to an object and receive a response signal from the object; a detector configured to detect one or more successive sensor signals, wherein the one or more sensor signals each comprise at least one of location information representing a three-dimensional location of the probe and orientation information representing a directivity of the probe on the object; a storage unit configured to store the at least one of the sensor signals and at least one or more data signals corresponding respectively to the one or more sensor signals, wherein the at least one or more data signals are acquired based on the response signal; and an image processor configured to form one or more display images by using the one or more sensor signals and the one or more data signals stored in the storage unit, wherein each of the one or more display images includes an image indicator formed based on a corresponding sensor signal among the one or more sensor signals and an ultrasound image formed based on a corresponding data signal among the one or more data signals.

According to another aspect of the present disclosure, there is provided an ultrasound diagnosis apparatus including: a probe configured to transmit an ultrasound signal to an object and receive a response signal from the object; a detector configured to detect a sensor signal including at least one of location information representing a three-dimensional location of the probe and orientation information representing a directivity of the probe on the object; a storage unit configured to store the sensor signal associated with the response signal; a first former configured to form an ultrasound image based on the received response signal; a second former configured to form an image indicator based on the sensor signal stored in the storage unit; and a third former configured to form a display image including the ultrasound image and an image indicator corresponding to the ultrasound image.

### BRIEF DESCRIPTION OF THE DRAWINGS

The above and other features and advantages of the present disclosure will become more apparent by describing in detail exemplary embodiments thereof with reference to the attached drawings in which:
FIG. 1 is a block diagram of an ultrasound diagnosis apparatus according to an embodiment of the present disclosure;
FIG. 2 is a flowchart illustrating an operating method of the ultrasound diagnosis apparatus shown in FIG. 1;
FIG. 3 is a diagram showing an example of a display image output on an output unit shown in FIG. 1;
FIG. 4 is a diagram showing an example of an image indicator included in a display image;
FIG. 5 is a diagram showing an example of relations between N frame data signals and M sensor sub-signals;
FIG. 6 is a diagram showing an example of a method of forming a display image in an image processing unit shown in FIG. 1;
FIG. 7 is a diagram showing an example of a storage unit shown in FIG. 1;
FIG. 8 is a diagram showing another example of the storage unit shown in FIG. 1; and
FIG. 9 is a diagram showing an example of the image processor shown in FIG. 1.

### DETAILED DESCRIPTION

Hereinafter, embodiments of the present disclosure will be described with reference to the accompanying drawings. Expressions such as "at least one of," when preceding a list of elements, modify the entire list of elements and do not modify the individual elements of the list.

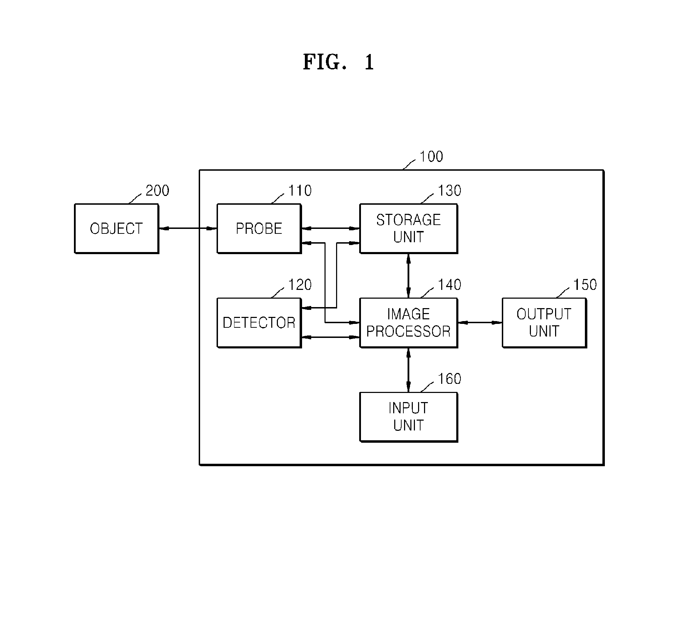

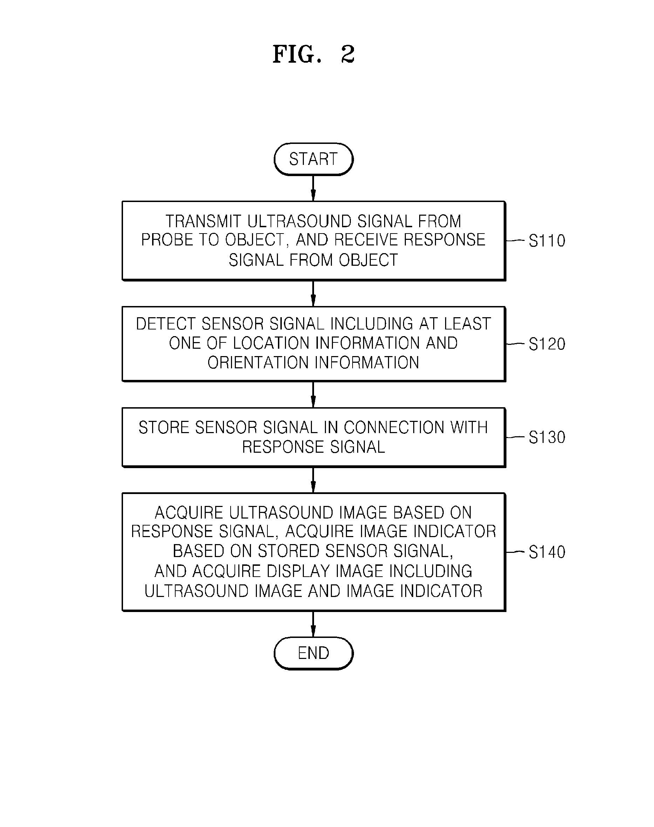

FIG. 1 is a block diagram of an ultrasound diagnosis apparatus 100 according to an embodiment of the present disclosure, and FIG. 2 is a flowchart illustrating an operating method of the ultrasound diagnosis apparatus of FIG. 1.

Referring to FIGS. 1 and 2, the ultrasound diagnosis apparatus 100 includes a probe 110, a detector 120, a storage unit 130, and an image processor 140. The ultrasound diagnosis apparatus 100 may further include an output unit 150 and an input unit 160.

In an exemplary method for operating the ultrasound diagnosis apparatus 100, the probe 110 may transmit an ultrasound signal to an object 200, and receive a response signal (S110). The probe 110 may scan the object 200 through the transmission of the ultrasound signal and the receipt of the response signal. The response signal may be a signal that transmits through the object 200 or is reflected by the object 200.

The object 200 may be an animal body, such as a human body, or a part of an animal body. For example, the object 200 may be a fetus or an organ of an animal body.

The detector 120 acquires a sensor signal including at least one of location information representing a three-dimensional location of the probe 110, and orientation information representing a directivity of the probe 110 on the object 200 (S120). The location information may include three-dimensional coordinate values x, y, and z, and the orientation information may include a three-dimensional angle. In the location information, an origin may be an initial location of the probe 110 or the object 200, and in the orientation information, an origin may be the object 200.

The storage unit 130 may store the sensor signal acquired by the detector 120 associated with the response signal (S130).

The image processor 140 may form an ultrasound image based on the received response signal, form an image indicator based on the sensor signal stored in the storage unit 130, and form a display image including the ultrasound image and the image indicator corresponding to the ultrasound image (S140).

The output unit 150 may output the display image. For example, the output unit 150 may be a display apparatus or a printer.

The storage unit 130 may further store a data signal obtained based on the response signal. The data signal may be processed by the image processor 140. The image processor 140 may form the ultrasound image by using the data signal stored in the storage unit 130, and may form the image indicator by using the sensor signal stored in the storage unit 130.

The input unit 160 may receive a user input from a user, and may transfer the user input to the image processor 140. The user input may include an output request for outputting the data signal stored in the storage unit 130. When receiving the output request from the input unit 160, the image processor 140 may form the display image based on the data signal and the sensor signal stored in the storage unit 130.

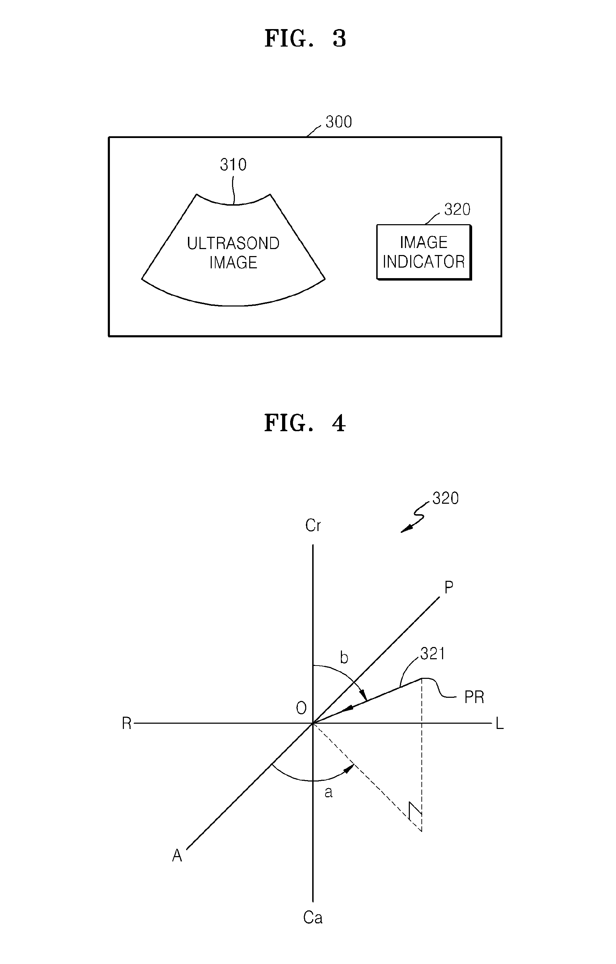

FIG. 3 is a diagram showing an example of a display image 300 output to the output unit 150 shown in FIG. 1.

Referring to FIGS. 1 and 3, the display image 300 output to the output unit 150 may include an ultrasound image 310 and an image indicator 320.

In the display image 300 of FIG. 3, the ultrasound image 310 and the image indicator 320 are separated from each other; however, the image of FIG. 3 is just an example of the display image 300. In some examples, the image indicator 320 may lie over the ultrasound image 310, unlike the example shown in FIG. 3.

FIG. 4 is a diagram showing an example of the image indicator 320 included in the display image 300.

Referring to FIGS. 3 and 4, the image indicator 320 may be a marker representing location information or orientation information of the object 200 by using one or more of an image and a test. The image indicator 320 may include a coordinate system consisting of anatomical axes [Cr(cranial)-Ca(caudal), A(anterior)-P(posterior), and R(right)-L(left)], and an arrow 321 may represent the location information or the orientation information. The center of the object may be located at the origion O of the coordinate system, and the probe 110 may be located at the end PR of the arraw 321. In addition, the image indicator 320 may further include text (a, b) representing an angle between the anatomical axis (Cr-Ca, A-P, and R-L) and the probe 110.

However, embodiments of the present disclosure are not limited to the example of the image indicator 320 shown in FIG. 4. The image indicator 320 may represent the location information or the orientation information of the probe 110 with respect to the object 200 in various ways.

Referring back to FIG. 1, the detector 120 may detect the location information or the orientation information of the probe 110 during a scanning session in which the probe 110 scans the object 200. During the scanning session, a three-dimensional location or orientation of the probe 110 may be fixed or may be movable.

Therefore, the detector 120 may detect one or more continuous sensor signals. Here, the one or more sensor signals may include at least one of the location information and the orientation information of the probe 110.

The storage unit 130 may store the one or more sensor signals and one or more data signals respectively corresponding to the one or more sensor signals. The one or more data signals may be acquired based on the response signal.

The image processor 140 may acquire one or more display images by using the one or more sensor signals and the one or more data signals stored in the storage unit 130. If there are a plurality of display images, the display images may represent moving pictures. Each of the one or more display images may include an image indicator 320 formed based on a corresponding sensor signal among the one or more sensor signals, and an ultrasound image 310 formed based on a corresponding data signal among the one or more data signals.

Otherwise, the detector 120 may detect M pieces of location information or M pieces of orientation information during the scanning session. Here, M is a positive natural number. In this case, the sensor signal may include M sensor sub-signals about the M pieces of location information or orientation information.

Data signals of N frames may be acquired based on the response signal acquired by the probe 110 during the scanning session. Here, N is a natural number. In this case, the data signals may include N frame data signals about the N frames. An n-th frame data signal is a signal for outputting an n-th ultrasound image about an n-th frame. For example, the n-th frame data signal may include a plurality of pixel values. In the present disclosure, n may be any positive natural number from 1 to N, (e.g., n=1, 2,..., N).

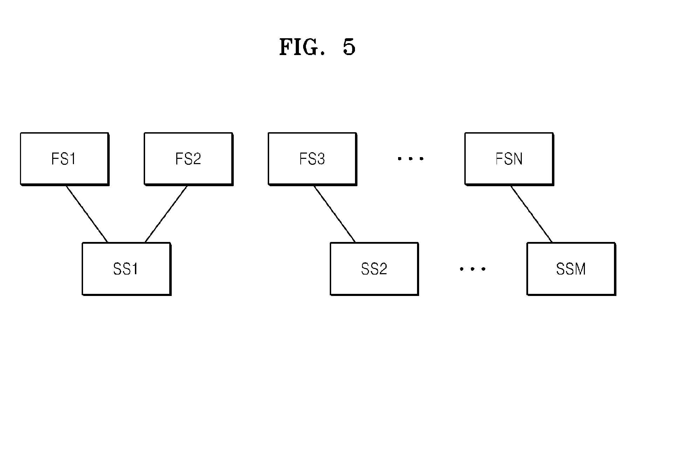

FIG. 5 is a diagram showing an example of relations between the N frame data signals and the M sensor sub-signals.

Referring to FIG. 5, each of the N frame data signals FS1, FS2, ..., FSN may correspond to one matching sensor sub-signal among M sensor sub-signals SS1, SS2, ..., SSM. Each of the M sensor sub-signals SS1, SS2, ..., SSM may correspond to one or more matching frames among the N frames.

The N frame data signals FS1, FS2, ..., FSN and the M sensor sub-signals SS1, SS2, ..., SSM may be acquired independently from each other during the scanning session. In this case, correspondence relations between the N frame data signals FS1, FS2, ..., FSN and the M sensor sub-signals SS1, SS2, ..., SSM may be determined based on times of acquiring the N frame data signals FS1, FS2, ..., FSN and times of acquiring the M sensor sub-signals SS1, SS2, ..., SSM.

In FIG. 5, the first frame representing data signal FS1 and the second frame representing data signal FS2 correspond to the first sensor sub-signal SS1. That is, the first sensor sub-signal SS1 is the matching sensor sub-signal for the first frame and for the second frame, and the first and second frames are matching frames of the first sensor sub-signal SS1.

In FIG. 5, the N frame data signals FS1, FS2, ..., FSN and the M sensor sub-signals SS1, SS2, ..., SSM correspond to each other in a many-to-one correspondence, where many may mean greater than one. However, FIG. 5 shows only an example of the correspondence relation, and the N frame data signals FS1, FS2, ..., FSN and the M sensor sub-signals may correspond to each other in various manners, for example, a one-to-one correspondence, a many-to-one correspondence, or a one-to-many correspondence.

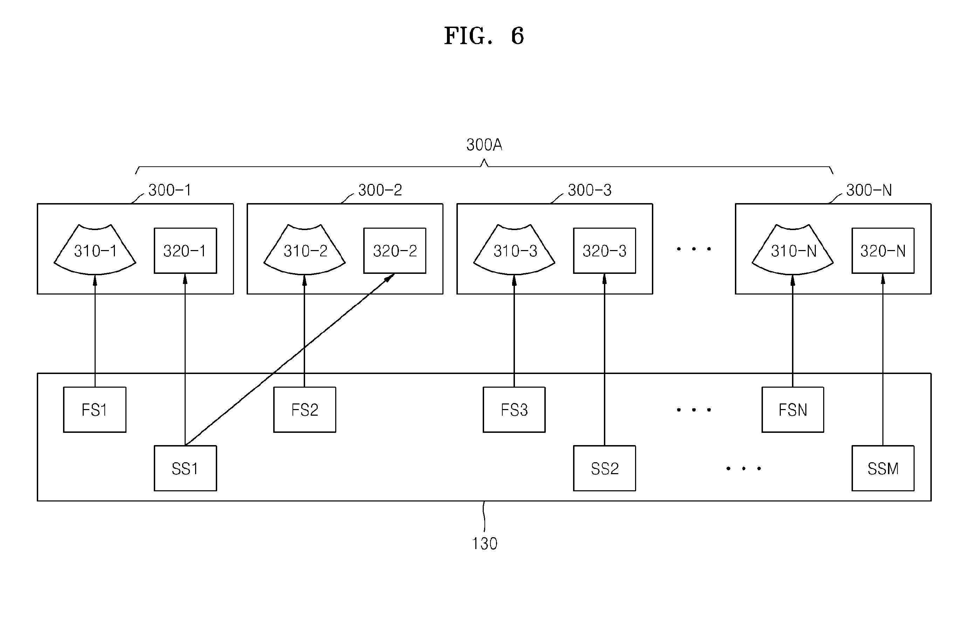

FIG. 6 is a diagram showing an example of forming a display image 300A in the image processor 140 shown in FIG. 1.

Referring to FIGS. 1 and 6, the display image 300A formed by the image processor 140 may include N frame display images 300-1, 300-2,..., 300-N of N frames. For example, an n-th frame display image of an n-th frame may include an n-th frame ultrasound image 310-n (not shown) and an n-th frame image indicator 320-n (not shown).

The storage unit 130 may store the N frame data signals FS1, FS2, ..., FSN and M sensor sub-signals SS1, SS2, ..., SSM. In FIG. 6, the correspondence relation between the N frame data signals FS1, FS2, ..., FSN and the M sensor sub-signals SS1, SS2, ..., SSM is the same as that shown in FIG. 5. However, the application is not limited to the correspondence shown in FIGS. 5 and 6, and other examples may have different correspondence.

The image processor 140 may form an n-th frame ultrasound image 310-n based on the n-th frame data signal FSn stored in the storage unit 130. The image processor 140 may form an n-th frame image indicator 320-n based on the matching sensor sub-signal of the n-th frame among the M sensor sub-signals SS1, SS2, ..., SSM stored in the storage unit 130. For example, the third frame image indicator 320-3 may be formed based on the second sensor sub-signal SS2, that is, the matching sensor sub-signal of the third frame.

In order for the image processor 140 to identify the matching sensor sub-signal of the n-th frame among the M sensor sub-signals SS1, SS2, ..., SSM, the storage unit 130 may further store relational information representing the correspondence relation between the N frame data signals FS1, FS2, ..., FSN and the M sensor sub-signals SS1, SS2, ..., SSM.

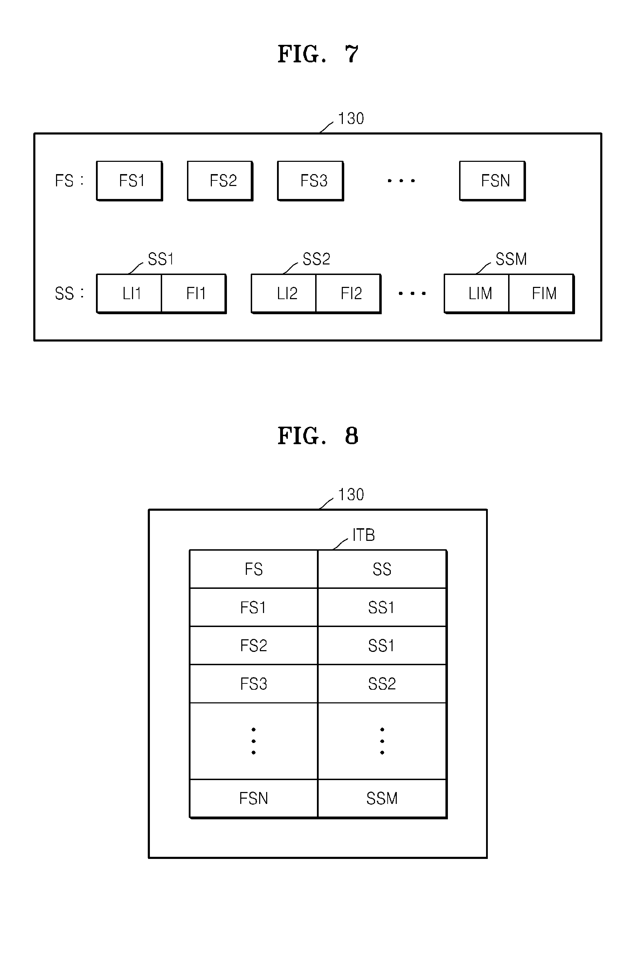

FIG. 7 is a diagram showing an example of the storage unit 130 shown in FIG. 1.

Referring to FIGS. 1 and 7, the storage unit 130 may store the N frame data signals FS1, FS2, ..., FSN included in a data signal FS and the M sensor sub-signals SS1, SS2, ..., SSM included in a sensor signal SS.

Each of the M sensor sub-signals SS1, SS2, ..., SSM may include sensor information LI1, LI2, ..., LIM and frame information FI1, FI2, ..., FIM. The sensor information LI1, LI2, ..., LIM indicates at least one of the location information and the orientation information of the probe 110. Each piece of the frame information FI1, FI2, ..., FIM indicates the matching frame of a corresponding sensor sub-signal.

When the correspondence relation between the N frame data signals FS1, FS2, ..., FSN and the M sensor sub-signals SS1, SS2, ..., SSM in FIG. 7 is the same as that of FIG. 5, the frame information FI1 included in the first sensor sub-signal SS1 may indicate the first and second frames.

FIG. 8 is a diagram showing another example of the storage unit 130 shown in FIG. 1.

Referring to FIGS. 1 and 8, the storage unit 130 may store the N frame data signals FS1, FS2, ..., FSN, the M sensor sub-signals SS1, SS2, ..., SSM, and an information table ITB indicating relational information FS-SS.

However, a method of storing the N frame data signals FS1, FS2, ..., FSN, the M sensor sub-signals SS1, SS2, ..., SSM, and the relational information FS-SS in the storage unit 130 is not limited to the examples shown in FIGS. 7 and 8.

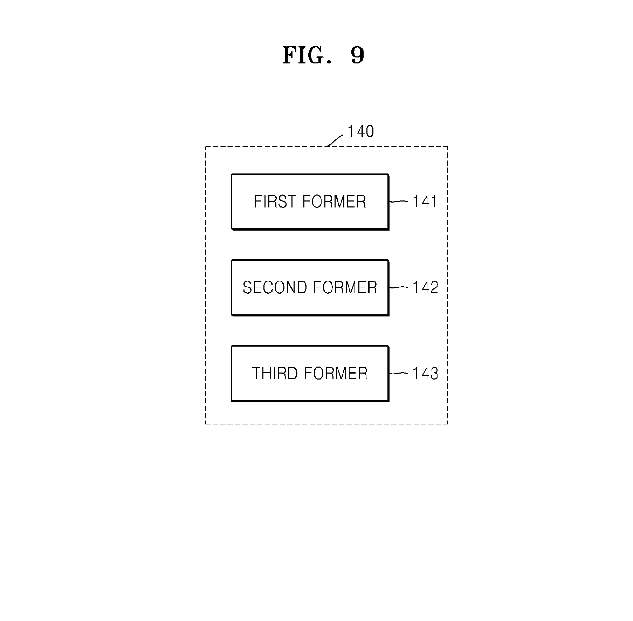

FIG. 9 is a diagram showing an example of the image processor 140 of FIG. 1.

Referring to FIGS. 1 and 9, the image processor 140 may include a first former 141, a second former 142, and a third former 143. The first former 141 may form an ultrasound image 310 based on the response signal received by probe 110. The second former 142 may form an image indicator 320 based on the sensor signal stored in the storage unit 130. The third former 143 may form a display image including the ultrasound image 310 and the image indicator 320 corresponding to the ultrasound image 310 (see, FIG. 3).

FIG. 9 is an example of the implementation of the image processor 140. In another example, each of the first former 141, the second former 142, and the third former 143 is an independent processor. Alternatively, the second former 142, and the third former 143 are implemented as one processor.

As described above, according to the embodiments of the present disclosure, the ultrasound diagnosis apparatus and the operating method of the ultrasound diagnosis apparatus may be provided so that the user of the ultrasound diagnosis apparatus may intuitively recognize the state of scanning the object.

The ultrasound diagnosis apparatus may automatically store the sensor signals including the sensor information indicating the three-dimensional location or orientation of the probe with respect to the object in the storage unit while the probe is scanning the object. Therefore, the user does not need to manipulate the ultrasound diagnosis apparatus while scanning the object, in order to display the image indicator on the ultrasound image. Thus, operating property, efficiency, and productivity may be improved.

When the ultrasound image is output based on the data signal stored in the storage unit, the image indicator is output with the ultrasound image based on the sensor signal stored in the storage unit. Therefore, the user may intuitively recognize the scanning manner of the object through the image indicator.

In addition, when a person who performs the scanning of the object and a person who analyzes the ultrasound image are different from each other, communication between the scanning person and the analyzing person may be performed easily and clearly.

According to the embodiments of the present disclosure, the ultrasound diagnosis apparatus and the operating method of the ultrasound diagnosis apparatus may be provided so that the user of the ultrasound diagnosis apparatus may intuitively recognize the state of scanning the object.

The embodiments of the present disclosure can be written as computer programs and can be implemented in general-use digital computers or computer processors that execute the programs using a non-transitory computer readable recording medium. Examples of the computer readable recording medium include magnetic storage media (e.g., ROM, RAM, USB, floppy disks, hard disks, etc.), optical recording media (e.g., CD-ROMs, and DVDs), and PC interfaces (e.g., PCI, PCI-express, Wifi, etc.).

While the present disclosure has been particularly shown and described with reference to exemplary embodiments thereof, it will be understood by those of ordinary skill in the art that various changes in form and details may be made therein without departing from the spirit and scope of the present disclosure as defined by the following claims.

## Claims

1. An ultrasound diagnosis apparatus comprising:
a probe configured to transmit an ultrasound signal to an object and receive a response signal from the object;
a detector configured to detect a sensor signal including at least one of location information representing a three-dimensional location of the probe and orientation information representing a directivity of the probe on the object;
a storage unit configured to store the sensor signal associated with the response signal; and
an image processor configured to form an ultrasound image based on the received response signal, form an image indicator based on the sensor signal stored in the storage unit, and form a display image including the ultrasound image and the image indicator corresponding to the ultrasound image.

2. The ultrasound diagnosis apparatus of claim 1, further comprising an output unit configured to output the display image.

3. The ultrasound diagnosis apparatus of claim 1, wherein the location information represents three-dimensional coordinate values x, y, and z, and the orientation information represents a three-dimensional angle.

4. The ultrasound diagnosis apparatus of claim 1, wherein the storage unit is further configured to store a data signal acquired based on the response signal, and the image processor is further configured to form the ultrasound image by using the data signal stored in the storage unit and form the image indicator by using the sensor signal stored in the storage unit.

5. The ultrasound diagnosis apparatus of claim 4, wherein the data signal comprises N frame data signals about N frames and the sensor signal comprises M sensor sub-signals, each of the N frame data signals corresponds to one of the M sensor sub-signals, and N and M are positive natural numbers.

6. The ultrasound diagnosis apparatus of claim 5, wherein the display image comprises N frame display images of the N frames, and an n-th frame display image of an n-th frame among the N frame display images comprises an n-th frame ultrasound image and an n-th frame image indicator, wherein n is a positive natural number among 1 to N.

7. The ultrasound diagnosis apparatus of claim 6, wherein the image processor is further configured to form the n-th frame ultrasound image based on the n-th frame data signal stored in the storage unit, and form the n-th frame image indicator based on a sensor sub-signal corresponding to the n-th frame data signal among the M sensor sub-signals stored in the storage unit.

8. The ultrasound diagnosis apparatus of claim 7, wherein the storage unit is further configured to store relational information indicating correspondence relations between the N frame data signals and the M sensor sub-signals.

9. The ultrasound diagnosis apparatus of claim 8, wherein the relational information may be frame information included in each of the M sensor sub-signals, or the storage unit is configured to store the N frame data signals, the M sensor sub-signals, and an information table indicating the relational information.

10. The ultrasound diagnosis apparatus of claim 1, wherein the image indicator is a marker representing the location information and the orientation information of the probe with respect to the object by using at least one of an image and a text.

11. The ultrasound diagnosis apparatus of claim 1, wherein the image indicator lies over the ultrasound image.

12. A method of operating an ultrasound diagnosis apparatus, the method comprising:
transmitting an ultrasound signal from a probe to an object, and receiving a response signal from the object;
detecting a sensor signal including at least one of location information representing a three-dimensional location of the probe and orientation information representing a directivity of the probe on the object;
storing the sensor signal associated with the response signal; and
forming an ultrasound image based on the received response signal, forming an image indicator based on the stored sensor signal, and forming a display image including the ultrasound image and the image indicator.

13. The method of claim 12, further comprising outputting the display image.

14. The method of claim 12, wherein the location information represents three-dimensional coordinate values x, y, and z, and the orientation information represents a three-dimensional angle.

15. The method of claim 12, further comprising storing a data signal acquired based on the response signal, wherein in the step of forming the display image, the ultrasound image is formed by using the stored data signal and the image indicator is formed by using the stored sensor signal.

16. The method of claim 15, wherein the data signal comprises N frame data signals about N frames and the sensor signal comprises M sensor sub-signals, each of the N frame data signals corresponds to one of the M sensor sub-signals, and N and M are positive natural numbers.

17. The method of claim 16, wherein the display image comprises N frame display images of the N frames, and an n-th frame display image of an n-th frame among the N frame display images comprises an n-th frame ultrasound image and an n-th frame image indicator, wherein n is a positive natural number among 1 to N.

18. The method of claim 17, wherein the step of forming the ultrasound image further comprises forming the n-th frame ultrasound image based on the stored n-th frame data signal, and the step of forming of the image indicator comprises forming the n-th frame image indicator based on a sensor sub-signal corresponding to the n-th frame data signal among the stored M sensor sub-signals.

19. The method of claim 12, wherein the image indicator is formed to lie over the ultrasound image.

20. An ultrasound diagnosis apparatus comprising:
a probe configured to transmit an ultrasound signal to an object and receive a response signal from the object;
a detector configured to detect at least one of location information representing a three-dimensional location of the probe and orientation information representing a directivity of the probe on the object; and
a storage unit configured to store the at least one of the location information and the orientation information associated with the received response signal.

21. A method of operating an ultrasound diagnosis apparatus, the method comprising:
transmitting an ultrasound signal from a probe to an object, and receiving a response signal from the object;
detecting at least one of location information representing a three-dimensional location of the probe and orientation information representing a directivity of the probe on the object; and
storing the at least one of the location information and the orientation information associated with the response signal.

22. A non-transitory computer readable recording medium containing a program that, when executed, cause one or more computer processors to implement the method of operating the ultrasound diagnosis apparatus of any one of claims 12 to 19 and 21.

23. An ultrasound diagnosis apparatus comprising:
a probe configured to transmit an ultrasound signal to an object and receive a response signal from the object;
a detector configured to detect one or more successive sensor signals, wherein the one or more sensor signals each comprise at least one of location information representing a three-dimensional location of the probe and orientation information representing a directivity of the probe on the object;
a storage unit configured to store the at least one of the sensor signals and at least one or more data signals respectively corresponding to the one or more sensor signals, wherein the at least one or more data signals are acquired based on the response signal; and
an image processor configured to form one or more display images by using the one or more sensor signals and the one or more data signals stored in the storage unit, wherein each of the one or more display images comprises an image indicator formed based on a corresponding sensor signal among the one or more sensor signals and an ultrasound image formed based on a corresponding data signal among the one or more data signals.

24. An ultrasound diagnosis apparatus comprising:
a probe configured to transmit an ultrasound signal to an object and receive a response signal from the object;
a detector configured to detect a sensor signal including at least one of location information representing a three-dimensional location of the probe and orientation information representing a directivity of the probe on the object;
a storage unit configured to store the sensor signal associated with the response signal;
a first former configured to form an ultrasound image based on the received response signal;
a second former configured to form an image indicator based on the sensor signal stored in the storage unit; and
a third former configured to form a display image including the ultrasound image and the image indicator corresponding to the ultrasound image.

25. The ultrasound diagnosis apparatus of claim 24, wherein the first former, the second former and the third former are included in an image processor.
